# EUROPÄISCHE PATENTSCHRIFT

(11) **EP 0 322 687 B1**
(45) Veröffentlichungstag und Bekanntmachung des Hinweises auf die Patenterteilung: **05.05.1993**
(21) Anmeldenummer: 88121172.6
(22) Anmeldetag: 17.12.1988
(51) Int. Cl.: A61K 9/26, B01D 11/00

(54) **Verfahren zur Herstellung einer mindestens einen Wirkstoff und einen Träger umfassenden Zubereitung**
Method for the manufacture of a product having at least one substance embedded in a carrier
Procédé de préparation d'un produit ayant au moins un agent thérapeutique encastré dans véhicule

(30) Priorität: 28.12.1987 DE 3744329
(43) Veröffentlichungstag der Anmeldung: 05.07.1989
(73) Patentinhaber: SCHWARZ PHARMA AG, D-40789 Monheim/Rhld. (DE)
(72) Erfinder: Fischer, Wilfried, Dr. rer. nat., D-5093 Burscheid (DE); Müller, Bernd W., Prof. Dr., D-2302 Flintbek (DE)

(56) Entgegenhaltungen:
- EP-A- 0 088 405
- WO-A-87/02697
- DE-A- 2 450 978
- DE-A- 3 323 940

## Beschreibung

Die Erfindung betrifft ein Verfahren zur Herstellung einer einen Wirkstoff und einen Träger umfassenden Arzneiform.

Die effektive Langzeitanwendung von Arzneistoffen wird u.a. durch eine häufige Applikation der Arzneiform und die sich hieraus ergebenden Complianceprobleme beim Patienten begrenzt. Eine sich über Monate oder Jahre erstreckende ein- oder mehrmalige tägliche Medikamenteneinnahme führt auf die Dauer zu einer unzuverlässigen Einnahme und damit unzuverlässigen Wirkung.

Arzneistoffe, die nach oraler Applikation in geringem Ausmaß absorbiert werden oder die Haut oder Schleimhäute nur unzureichend penetrieren können, müssen injiziert werden. Bei einer Langzeitanwendung der betreffenden Medikamente ist eine mehrmalige tägliche Injektion für den Patienten unzumutbar, da sie häufig schmerzhaft ist.

Ferner gibt es eine große Zahl von Arzneistoffen, die sich im Applikationsbereich zersetzen oder metabolisiert werden und deren pharmakokinetisches Profil nicht befriedigt.

Literaturbekannt sind Arzneiformen, die aus einer Polymermatrix bestehen, in die ein Arzneistoff eingebracht worden ist, der aus dieser Arzneiform retardiert freigesetzt wird. Diese Polymereinbettungen können Injektionsarzneiformen oder Implantationsarzneiformen sein. So beschreibt beispielsweise die DE-OS 3 428 372 die Einbettung von regulatorischen Peptiden in Poly-D(-)-3-hydroxybuttersäure und die EP-OS 0 052 510 die Einbettung von LH-RH in Polymermaterialien, beispielsweise Polymilchsäure oder Polymilchsäure/Polyglykolsäurecopolymere.

In den Körper als subcutane Injektion oder Implantation eingebracht, lösen sich die Einbettungen langsam auf und setzen den Wirkstoff über mehrere Tage oder sogar Monate frei. Mit Arzneiformen dieser Art kann die Injektionsfrequenz drastisch gesenkt und damit die Compliance und Effektivität des Arzneimittels erhöht werden.

Die Herstellung solcher Retardarzneiformen geschieht nach folgenden Vefahren:
- Mikroverkapselung mit organischen Lösungsmitteln
   (L.M. Sanders et al., J. Contr. Release, 2 (1985) 187 oder P.B. Deasy, Microencapsulation and Related Drug Processes, M. Dekker Inc., New York, 1984);
- Emulgierung und anschließende Lösungsmittelverdampfung
   (T.R. Tice & R.M. Gilley, J. Contr. Release, 2, (1985) 343;
- Sprühtrocknung
   (D.L. Wise et al., Life Sci., 19, (1976) 867;
- Extrusion
   (A.J. Schwope et al., Life Sci., 17, (1975) 1877;
- Schmelzeinbettung
   (A.J. Schwope et al., Life Sci., 17, (1975) 1877; und
- Grenzflächenpolymerisation
   (G.Birrenbach & P. Speiser, J. Pharm. Sci., 65, (1976) 1763.

Die genannten Verfahren sind jedoch entweder mit dem Nachteil behaftet, daß sie mit großen Mengen toxischer organischer Lösungsmittel arbeiten, wobei die resultierenden Polymereinbettungen hohe Lösungsmittelrestkonzentrationen aufweisen; vgl. J.P. Benoit et al., Int. J. Pharmaceutics, 29 (1986) 95. Oder die genannten Verfahren arbeiten bei hohen Temperaturen oder Drucken, die insbesondere zu hohen lokalen Temperaturerhöhungen führen und die inkorporierten Arzneistoffe schädigen können; vgl. L.M. Sanders et al., J. Pharm. Sci., 75 (1986) 356. Wenn eine Arzneiform über längere Zeit unter der Haut oder im Gewebe verbleibt, sind von organischen Lösungsmitteln lokal toxische Gewebereaktionen zu erwarten. Aus diesem Grund müssen Lösungsmittelrestmengen soweit wie möglich aus den gewünschten Produkten entfernt werden.

Neben den Lösungsmitteln in der Polymermatrix findet man bei durch Emulsionsverfahren hergestellten Mikropartikeln etwa 1 bis 2 % des Dispersionsmittels an der Oberfläche adsorbiert. Das Lösungsmittel kann durch Waschprozesse nicht entfernt werden. In der Regel wird Silikonöl oder eine andere lipophile Flüssigkeit verwendet, die den Nachteil aufweist, durch den Organismus nicht abbaubar zu sein; - EP-OS 81.305 426.9 -.

Ferner enthalten alle durch Polymerisation hergestellten Mikropartikel (beispielsweise Nanokapseln) in der Polymermatrix sowohl Mono-, Di- und Oligomere als auch häufig Initiatormoleküle. Alle diese Substanzen verleihen den Mikropartikeln erheblich schlechter zu bewertende toxikologische Eigenschaften.

Der Grenzflächenpolymerisation und auch dem Emulsionsverfahren haftet als wesentlicher Nachteil an, daß eine Überführung in technischem Maßstab bis zum heutigen Tage nicht oder nur unbefriedigend gelungen ist, so daß die Einführung entsprechender Präparate bisher ausgeblieben ist; daran konnte auch das sogenannte Nanokapselpatent (Speiser DE 29 65 725) nichts ändern.

Schließlich arbeiten viele Verfahren, die sich organischer Lösungsmittel bedienen, nicht in geschlossenen Systemen. Die dadurch verursachte Umweltbelastung ist erheblich. Die erforderliche Reinigung der Luft durch Rückgewinnungsanlagen ist teuer und bei großen Durchsätzen, wie sie z.B. bei der Sprühtrocknung üblich sind, technisch problematisch.

Eine unabdingbare Forderung, die an o.g. Wirkstoffeinbettungen zu stellen ist, ist die Sterilität der Formulierungen. Mit den vorstehend genannten Verfahren resultieren per se keine sterilen Arzneiformen. Entweder muß der gesamte Herstellungsprozeß mit großem Aufwand unter sterilen oder aseptischen Bedingungen durchgeführt werden oder die Arzneiform muß nach ihrer Herstellung sterilisiert werden. Zur Zeit ist eine Nachsterilisation durch Bestrahlen mit Gammastrahlen üblich. Abgesehen von den behördlichen Bestimmungen, die Arzneimittel, die mit Gammastrahlen sterilisiert worden sind, nur unter ganz bestimmten Bedingungen in den Handel gelangen zu lassen, sind mit dieser Sterilisationsart Schäden für das Arzneimittel verbunden, insbesondere wenn es sich um Peptidarzneistoffe handelt. Auch die Polymermatrix, beispielsweise Polymilchsäure, kann durch Gammastrahlen zersetzt werden. Hierdurch kann sich das ursprüngliche Freisetzungs- oder Auflöseverhalten der Arzneiform in unkontrollierter Weise ändern; vgl. (L.T. Sanders et al., J. Contr. Release, 2, (1985) 187.

Aufgabe der vorliegenden Erfindung ist es nun, ein Verfahren zur Herstellung einer einen Wirkstoff oder Wirkstoffe und einen Träger oder Träger umfassenden Arzneiform vorzusehen, welches Lösungsmittelrestmengen vermeidet oder zumindest auf toxikologisch unbedenkliche Mengen reduziert.

Ferner ist es Aufgabe der Erfindung, ein Verfahren zur Herstellung einer einen Wirkstoff oder Wirkstoffe und ein Polymeres oder Polymere als Träger umfassenden Arzneiform vorzusehen, die frei von Mono-, Di- und Oligomeren sowie von Initiatormolekülen ist.

Weitere Aufgabe der Erfindung ist es, ein Verfahren zur Herstellung einer einen Wirkstoff oder Wirkstoffe und einen Träger oder Träger umfassenden Arzneiform vorzusehen, bei dem die Sterilisation während des Verfahrens in gesetzlich uneingeschränkt erlaubter und für die Produktqualität unbedenklicher Weise erfolgt.

Schließlich ist es Aufgabe der Erfindung, eine Vorrichtung vorzusehen, mit der das erfindungsgemäße Verfahren durchgeführt werden kann.

Gemäß einer Ausführungsform sieht die Erfindung ein Verfahren zur Herstellung einer einen Wirkstoff oder Wirkstoffe und einen Träger oder Träger umfassenden Arzneiform vor, bei dem man
- ein flüssiges Medium enthaltend den Wirkstoff oder Wirkstoffe und den Träger oder die Träger mit einem fluiden Gas in Berührung bringt,
- das fluide Gas entspannt und
- die vom flüssigen Medium befreite Wirkstoff/Träger-Zubereitung gewinnt.

Bei diesem Verfahren kann man ein flüssiges Medium verwenden, in dem
- sowohl der/die Wirkstoff/e als auch der/die Träger gelöst oder dispergiert sind oder
- der/die Wirkstoff/e gelöst und der/die Träger dispergiert ist/sind oder
- der/die Wirkstoff/e dispergiert und der/die Träger gelöst ist/sind.

Man kann den/die Wirkstoff/e mit dem flüssigen Medium einerseits und den/die Träger mit dem flüssigem Medium andererseits getrennt mit dem fluiden Gas in Berührung bringen.

Gemäß einer weiteren Ausführungsform betrifft die Erfindung ein Verfahren zur Herstellung einer einen Wirkstoff und einen Träger umfassenden Arzneiform, bei dem man
- den/die Wirkstoff/e und den/die Träger mit einem fluiden Gas in Berührung bringt, in dem der/die Wirkstoff/e und/oder der/die Träger löslich ist/sind,
- das fluide Gas entspannt,
- die vom fluiden Medium befreite Wirkstoff/Träger-Zubereitung gewinnt.

Gemäß einer weiteren Ausführungsform betrifft die Erfindung ein Verfahren zur Herstellung einer einen Wirkstoff oder Wirkstoffe und einen Träger oder Träger umfassende Arzneiform, bei dem man
(1) den/die Wirkstoff/e mit einem fluiden Gas in Berührung bringt, in dem der/die Wirkstoff/e löslich ist/sind, und danach das fluide Gas mit einem flüssigen Medium, enthaltend den/die Träger, in Berührung bringt, oder
(2) den/die Träger mit einem fluiden Gas in Berührung bringt, in dem der/die Träger löslich ist/sind und danach das fluide Gas mit einem flüsigen Medium, enthaltend den Wirkstoff oder die Wirkstoffe, in Berührung bringt,
   - das fluide Gas entspannt,
   - die vom flüssigen Medium befreite Wirkstoff/Träger-Zubereitung gewinnt.

Beispiele für erfindungsgemäß verwendbare Träger sind Polymermaterialien. Selbstverständlich lassen sich nach dem erfindungsgemäßen Verfahren auch Wirkstoff/Träger/Hilfsmittel-Zubereitungen herstellen, wenn man zusätzlich zum Wirkstoff und Träger noch Hilfsmittel einsetzt, beispielsweise Tenside.

Beispiele für Wirkstoffe sind Arzneimittel, Toxine, Insektizide, Viren und Diagnostika. Zum Begriff des Arzneistoffs wird auf die US-PS 3,773,919 verwiesen.

Die Extraktion lipophiler Stoffe durch überkritische Gase ist ein seit langem in der Lebensmitteltechnologie und in der chemischen Technologie angewandtes Verfahren; vgl. G.M. Schneider, Extraction with Supercritical Gases, Verlag Chemie, Weinheim, 1980. Dieses Verfahren wird beispielsweise zur Entkoffeinierung von Kaffee, Extraktion von Aromastoffen aus Pflanzen, Entölung von Lecithin oder zur Reinigung von Polymeren eingesetzt; vgl. DE-OS 33 23 940. Zur Erhöhung der Extraktausbeute aus Pflanzenzellen werden die Zellen zuvor mit Hilfe eines überkritischen Gases nach Druckbehandlung durch plötzliche Entspannung aufgebrochen. Diesen Vorgang bezeichnet man als Cell-Cracking.

Überraschenderweise wurde nun gefunden, daß das durch das erfindungsgemäße Verfahren erhaltene Produkt nicht nur eine verlängerte Wirkstofffreisetzung besitzt, sondern auch vollkommen steril vorliegt. Es ist zwar bekannt, daß durch Extraktion mit überkritischen Gasen gewonnene Extrakte steril sind. Dieser Effekt ist so zu verstehen, daß der Extrakt über die Gasphase vom Extraktionsrückstand getrennt wird, so daß keine Mikroorganismen über die Gasphase in den Extrakt gelangen können.

Überraschend ist jedoch, daß beim erfindungsgemäßen Verfahren für die Wirkstoff/Träger-Arzneiform als Extraktionsrückstand eine vollständige Keimabtötung erreicht werden kann. Zumal nach dem derzeitigen Stand der Technik zwar eine Keimzahlreduktion und Entwesung, jedoch keine Sterilisation möglich erscheint (vgl. E. Stahl und G. Rau, Dtsch. Apoth. Ztg. 125, 1999 (1985); E. Stahl, G. Rau und H. Kaltwasser, Naturwissenschaften 72, 144 (1985)). Auch die Keimzahlreduktion wird ausdrücklich als zu unwirtschaftlich dargestellt, da man mit hohen Drucken (etwa 2500 bar) und entsprechend aufwendigen Maschinen arbeiten muß.

Die keimtötende Wirkung des hier beanspruchten Verfahrens konnte an zahlreichen Testkeimen, und zwar sowohl an Bakterien als auch Pilzen und Hefen bestätigt werden. So führt eine 30-minütige Einwirkung eines überkritischen Gases (CO₂, 40° C / 140 bar) zu einer Abtötung folgender Testkeime:

| | |
|---|---|
| Bacillus subtilis | Sporen |
| Bacillus stearothermophilus | |
| Escherichia coli | |
| Salmonella edinburgh | |
| Staphylococcus aureus | |
| Pseudomonas aeruginosa | |
| Candida albicans | |
| Aspergillus niger | |

Gemäß einer Ausführungsform der Erfindung wird schließlich eine Vorrichtung zur Durchführung des erfindungsgemäßen Verfahrens mit
- einer Flüssiggaspumpe,
- einem Sprühturm,
- einem Separator für die Wirkstoff/Träger-Zubereitung,
- ggf. einem Separator für das fakultative flüssige Medium und
- einem Kondensator für das Flüssiggas vorgesehen.

Als Düsen können Ein- oder Mehrstoffdüsen verwendet werden, bei denen das flüssige Medium entweder durch eine einzelne Öffnung oder durch eine Sinterscheibe hindurch zerstäubt wird. Bei den Einstoff-Düsen kann die Düsenöffnung kleiner 0,2 mm und bei Sinterdüsen etwa 10 m sein. Wenn man Zweistoffdüsen verwendet, kann man ein flüssiges Medium für den Wirkstoff und - getrennt davon - ein flüssiges Medium für den Träger vorsehen, die gemeinsam versprüht werden können.

Nachstehend wird die Erfindung durch zwei Abbildungen näher erläutert. Es zeigen:
Abb. 1 eine Vorrichtung zur Durchführung des Verfahrens gemäß der Erfindung und
Abb. 2 eine erfindungsgemäß erhaltene Wirkstoff/Träger-Arzneiform.

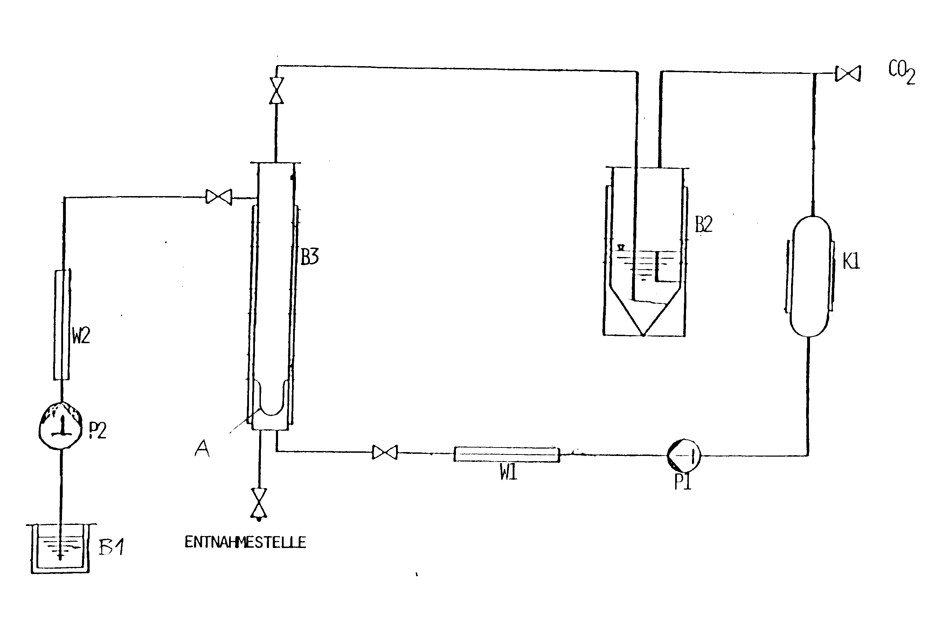

Eine erfindungsgemäße Vorrichtung kann nach Abb. 1 beispielsweise einen druckfesten Sprühturm (B3), eine Flüssiggaspumpe (P1), einen Wärmeaustauscher (W1), einen Separator (B2), eine Produktpumpe (P2) und einen Kondensator (K1) aufweisen. Das Flüssiggas kann durch den Wärmeaustauscher (W1) auf überkritische Bedingungen erhitzt werden und in dem Sprühturm (B3) dem versprühten Wirkstoff und dem versprühten Träger entgegenströmen, das flüssige Medium aufnehmen und danach in den Separator (B2) gelangen, wo das extrahierte flüssige Medium abgetrennt wird. Das fluide Gas wird danach in dem Kondensator (K1) kondensiert und über die Flüssiggaspumpe (P1) in den Kreislauf zurückgeführt. Die Wirkstoff/Träger-Zubereitung läßt sich am Boden des Separators (B2) steril entnehmen.

Eine weitere Ausführungsform der erfindungsgemäßen Vorrichtung kann nach Abbildung 1 beispielsweise einen druckfesten Sprühturm (B 3), eine Flüssiggaspumpe (P 1), einen Wärmeaustauscher (W 1), einen Separator (B 2), eine Produktpumpe (P 2) und einen Kondensator (K 1) aufweisen. Das Flüssiggas kann durch den Wärmeaustauscher (W 1) auf überkritische Bedingungen erhitzt werden und in den Sprühturm (B 3) mittels einer Mehrstoffdüse mit dem versprühten Wirkstoff und dem versprühten Träger im Gleichstrom oder mittels einer weiteren separaten Düse parallel zu dem aus der Mehrstoffdüse versprühten Wirkstoff und Träger im Gleichstrom versprüht werden, das flüssige Medium aufnehmen und danach in den Separator (B 2) gelangen, in dem das extrahierte flüssige Medium abgetrennt wird. Das fluide Gas wird danach in dem Kondensator (K 1) kondensiert und über die Flüssiggaspumpe (P 1) in den Kreislauf zurückgeführt. Die Wirkstoff/Träger-Zubereitung läßt sich am Boden des Separators (A) steril entnehmen.

Fluide Gase zeigen bei bestimmten Druck/Temperatur-Kombinationen Lösungsvermögen für Polymere, Lipide, Lecithine oder Wachse als Träger; vgl. F.P. Schmitz, J. Chromatography, 356, (1986) 261. Dementsprechend kann man die vorstehend beschriebene Vorrichtung in Fließrichtung vor dem Sprühturm B 3 um einen druckfesten Extraktor ergänzen und das fluide Gas mit dem/die Träger und dem/die Wirkstoff/e beladen und im Sprühturm B 3 über eine Düse gemeinsam als Einbettung abscheiden.

Selbstverständlich kann man auch das fluide Gas nur mit dem/den Wirkstoff/en oder nur mit dem/den Träger/Trägern beladen und den/die Träger bzw. den/die Wirkstoffe mit Hilfe der Produktpumpe P 2 dem Sprühturm B 3 zuführen. In diesem Fall erhält man eine Umhüllung oder ein Adsorbat.

Nachstehend wird die Erfindung durch zwei Beispiele näher erläutert.

### Beispiel 1 (Sterilisation)

Je 1 g d,l(+)-Polylactid werden mit definierten Mengen der folgenden Keime versetzt:

| Keimart | Konzentration (Keime/g) |
|---|---|
| Bacillus subtilis (Sporen) | 10², 10³, 1o⁴ |
| Bacillus stearothermophilus (Sporen) | 10², 10³, 10⁴ |
| E. coli | 10⁴ |
| Salmonella edinburgh | 10⁴ |
| Staphylococcus aureus | 10⁴ |
| Pseudomonas aeruginosa | 10⁴ |
| Candida albicans | 10⁴ |
| Aspergillus niger | 10⁴ |

Proben der kontaminierten Polymere werden mit CO₂ bei 140 bar und 50° C und 140 bar/40° C, für 12 h bzw. 0,5 h statisch behandelt. Danach wird in ca. 1 min entspannt; die Proben werden Sterilitätstests unterworfen.

### Ergebnis: alle Proben sind steril.

Parallel untersuchte, nicht mit CO₂ behandelte Proben zeigen, daß d,l(+)-Polylactid, MW 17.000 (PLA 17.000) selbst keine keimzahlreduzierenden Eigenschaften hat.

### Beispiel 2 (Sprüheinbettung)

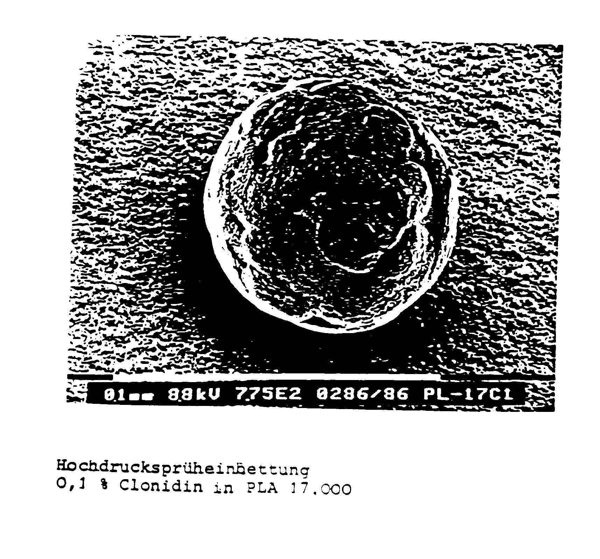

Eine 0,02-proz. Clonidin-HCl-Lösung in CH₂Cl₂, das 20 % PLA 17.000 enthält, wird mit 100 bar Überdruck in der Extraktionskolonne der Hochdruckanlage versprüht. Dabei wird CO₂ mit 100 bar/60° C im Gegenstrom in die Kolonne geleitet. Versprüht wird durch eine Düse aus Sintermetall (Porengröße ca. 5 bis 10 m). Nach wenigen cm Fallstrecke wird ein trockenes Pulver erhalten, das aus Hohlkugeln (ca. 100 m) besteht (Abb. 2). Die Hohlkugeln sind aus Untereinheiten zusammengesetzt, die vermutlich die bis 10 m großen Primärtröpfchen darstellen. Kurz nach Verlassen der Düse formen die Primärtröpfchen vermutlich die größeren Hohlkugeln.

### Wirkstofffreisetzung

Die Freisetzung des incorporierten Clonidin-HCl wurde über 24 h bei 37° C in Phosphatpuffer (pH = 7,5) verfolgt.

| Zeit (h) | Clonidin - HCl freigesetzt (%) |
|---|---|
| 1,5 | 40,5 |
| 5 | 48,8 |
| 24 | 52,0 |

### Methode: Hochdruckflüssigkeitschromatographie

Wie bei allen publizierten Wirkstofffreisetzungen aus PLA-Mikrokugeln wird oberflächlich adsorbiert bzw. in den oberen Schichten des Polymers eingelagerter Wirkstoff in der Initialphase schnell freigesetzt, während sich die Restdosis im Laufe mehrerer Tage (in diesem Fall ca. 10 Tage) aus der Einbettung herauslöst.

Eine Freisetzungsbeschleunigung ist durch Erhöhung des Beladungsgrades, Reduktion des Molekulargewichts oder der Einsatz der Oberflächeneigenschaften, durch Einsatz hydrophilerer Trägersubstanzen oder Einsatz hydrophiler Hilfsstoffe zu erreichen.

## Patentansprüche

1. Verfahren zur Herstellung einer einen Wirkstoff oder Wirkstoffe und einen Träger oder Träger umfassenden Arzneiform, dadurch gekennzeichnet, daß man
- ein flüssiges Medium, enthaltend den Wirkstoff oder Wirkstoffe und den Träger, oder die Träger mit einem fluiden Gas in Berührung bringt,
- die Flüssigkeit durch das fluide Gas entfernt,
- die vom flüssigen Medium befreite Wirkstoff/Träger-Zubereitung gewinnt.

2. Verfahren nach Anspruch 1, dadurch gekennzeichnet, daß man ein flüssiges Medium verwendet, in dem
- sowohl der/die Wirkstoff/e als auch der/die Träger gelöst oder dispergiert sind oder
- der/die Wirkstoff/e gelöst und der/die Träger dispergiert ist/sind oder
- der/die Wirkstoff/e dispergiert und der/die Träger gelöst sind.

3. Verfahren nach Anspruch 1, dadurch gekennzeichnet, daß man
- den/die Wirkstoff/e und den/die Träger mit einem fluiden Gas in Berührung bringt, in dem der/die Wirkstoff/e und/oder der/die Träger löslich ist/sind,
- das fluide Gas entspannt und
- die vom fluiden Gas befreite Wirkstoff/Träger-Zubereitung gewinnt.

4. Verfahren zur Herstellung einer einen Wirkstoff oder Wirkstoffe und einen Träger oder Träger umfassenden Arzneiform, dadurch gekennzeichnet, daß man
(1) den/die Wirkstoff/e mit einem fluiden Gas in Berührung bringt, in dem der/die Wirkstoff/e löslich ist/sind und danach das fluide Gas mit einem flüssigen Medium, enthaltend den/die Träger, in Berührung bringt, oder
(2) den/die Träger mit einem fluiden Gas in Berührung bringt, in dem der/die Träger löslich ist/sind und danach das fluide Gas mit einem flüssigen Medium, enthaltend den/die Wirkstoff/e, in Berührung bringt,
- das fluide Gas entspannt,
- die vom flüssigen Medium befreite Wirkstoff/Träger-Zubereitung gewinnt.

5. Verfahren nach einem der vorhergehenden Ansprüche dadurch gekennzeichnet, daß man Gase sowie niedrig siedende Flüssigkeiten und Mischungen davon, die sich in einen überkritischen Zustand überführen lassen, als fluides Gas verwendet.

6. Verfahren nach einem der vorhergehenden Ansprüche, dadurch gekennzeichnet, daß man als fluides Gas Distickstoffoxid, Kohlendioxid, Halogenkohlenwasserstoffe, Kohlenwasserstoffe (gesättigt und ungesättigt) Distickstoffdioxid oder Ammoniak oder Mischungen davon bzw. Mischungen mit Stickstoff und Schlepper verwendet.

7. Verfahren nach einem der vorhergehenden Ansprüche, dadurch gekennzeichnet, daß man einen oder mehrere Träger aus der Gruppe Polymere, Lipide, Lecithine und Wachse verwendet.

8. Verfahren nach einem der vorhergehenden Ansprüche, dadurch gekennzeichnet, daß man zusätzlich zu Wirkstoff oder Wirkstoffen und Träger noch Hilfsmittel, beispielsweise Tenside, einsetzt und eine Wirkstoff/-Träger/Hilfsmittel-Zubereitung gewinnt.

9. Verfahren nach einem der vorhergehenden Ansprüche, dadurch gekennzeichnet, daß man einen Arzneistoff, ein Toxin oder einen Virus bzw. Virusbruchstücke als Wirkstoff einsetzt.

10. Verfahren nach einem der vorhergehenden Ansprüche, dadurch gekennzeichnet, daß die Wirkstoff/Träger-Mischungen nach dem Behandeln mit dem fluiden Gas frei von vermehrungsfähigen Keimen sind.

11. Vorrichtung zur Durchführung des Verfahrens gemäß einem der vorhergehenden Ansprüche, gekennzeichnet durch
- eine Flüssiggaspumpe (P1),
- jeweils einen Verdampfer/Wärmetauscher (W1) und (W2),
- einen Sprühturm (B3),
- einen Separator für die Wirkstoff/Träger-Zubereitung (A),
- einen Separator (B2) für das fakultative flüssige Medium,
- einen Vorratsbehälter für das fakultative flüssige Medium (B1),
- einen Kondensator für das fluide Gas (K1).

## Claims

1. Process for the preparation of a drug form which comprises an active substance or active substances and a carrier or carriers, characterised in that
- a liquid medium containing the active substance or active substances and the carrier or the carriers is contacted with a fluid gas,
- the liquid is removed by the fluid gas,
- the active substance/carrier formulation freed of liquid medium is obtained.

2. Process according to Claim 1, characterised by use of a liquid medium in which
- both the active substance(s) and the carrier(s) are dissolved or dispersed or
- the active substance(s) is/are dissolved and the carrier(s) is/are dispersed or
- the active substance(s) is/are dispersed and the carrier(s) is/are dissolved.

3. Process according to Claim 1, characterised in that
- the active substance(s) and the carrier(s) are contacted with a fluid gas in which the active substance(s) and/or the carrier(s) is/are soluble,
- the pressure of the fluid gas is reduced and
- the active substance/carrier formulation freed of fluid gas is obtained.

4. Process for the preparation of a drug form comprising an active substance or active substances and a carrier or carriers, characterised in that
(1) the active substance(s) is/are contacted with a fluid gas in which the active substance(s) is/are soluble, and then the fluid gas is contacted with a liquid medium containing the carrier(s), or
(2) the carrier(s) is/are contacted with a fluid gas in which the carrier(s) is/are soluble, and then the fluid gas is contacted with a liquid medium containing the active substance(s),
- the pressure of the fluid gas is reduced,
- the active substance/carrier formulation freed of liquid medium is obtained.

5. Process according to any of the preceding claims, characterised in that gases and low-boiling liquids and mixtures thereof, which can be converted into a super-critical state, are used as fluid gas.

6. Process according to any of the preceding claims, characterised in that dinitrogen oxide, carbon dioxide, halohydrocarbons, hydrocarbons (saturated and unsaturated), dinitrogen dioxide or ammonia or mixtures thereof or mixtures with nitrogen and entrainer is/are used as fluid gas.

7. Process according to any of the preceding claims, characterised in that one or more carriers from the group comprising polymers, lipids, lecithins and waxes is/are used.

8. Process according to any of the preceding claims, characterised in that aids, for example surfactants, are also employed in addition to active substance or active substances and carrier, and an active substance/carrier/aid formulation is obtained.

9. Process according to any of the preceding claims, characterised in that a medicinal substance, a toxin or a virus or virus fragments is/are employed as active substance.

10. Process according to any of the preceding claims, characterised in that the active substance/carrier mixtures after the treatment with the fluid gas are free of microbes able to multiply.

11. Device for carrying out the process according to any of the preceding claims, characterised by
- a liquid gas pump (P1),
- in each case a vaporizer/heat exchanger (W1) and (W2),
- a spray tower (B3),
- a separator for the active substance/carrier formulation (A),
- a separator (B2) for the optional liquid medium,
- a storage container for the optional liquid medium (B1),
- a condenser for the fluid gas (K1).

## Revendications

1. Procède de préparation d'une forme médicamenteuse comprenant une ou des substances actives et un ou des supports, caractérisé en ce que
- on met en contact un milieu liquide, contenant la ou les substances actives et le ou les supports, avec un fluide gazeux,
- on élimine le liquide au moyen du fluide gazeux,
- on récupère la préparation substance active/support débarrassée du milieu liquide.

2. Procédé selon la revendication 1, caractérisé en ce qu'on utilise un milieu liquide dans lequel
- aussi bien la ou les substances actives que le ou les supports sont dissous ou dispersés, ou
- la ou les substances actives sont dissoutes et le ou les supports sont dispersés, ou
- la ou les substances actives sont dispersées et le ou les supports sont dissous.

3. Procédé selon la revendication 1, caractérisé en ce que
- on met en contact la ou les substances actives et le ou les supports avec un fluide gazeux, dans lequel la ou les substances actives et/ou le ou les supports sont solubles,
- on détend le fluide gazeux, et
- on récupère la préparation substance active/support débarrassée du fluide gazeux.

4. Procédé de préparation d'une forme médicamenteuse comprenant une ou des substances actives et un ou des supports, caractérisé en ce que
(1) on met en contact la ou les substances actives avec un fluide gazeux, dans lequel la ou les substances actives sont solubles, puis on met en contact le fluide gazeux avec un milieu liquide contenant le ou les supports, cou
(2) on met en contact le ou les supports avec un fluide gazeux dans lequel le ou les supports sont solubles, puis on met en contact le fluide gazeux avec un milieu liquide contenant la ou les substances actives,
- on détend le fluide gazeux,
- on obtient la préparation substance active/support débarrassée du milieu liquide.

5. Procédé selon l'une des revendications précédentes, caractérisé en ce qu'on utilise comme fluides gazeux des gaz ainsi que des liquides à bas point d'ébullition et des mélanges de ceux-ci, qui peuvent être amenés à un état sur-critique.

6. Procédé selon l'une des revendications précédentes, caractérisé en ce qu'on utilise comme fluide gazeux le protoxyde d'azote, l'anhydride carbonique, les hydrocarbures halogénés, les hydrocarbures (saturés et insaturés), l'oxyde d'azote ou l'ammoniaque ou des mélanges de ceux-ci ou des mélanges avec de l'azote et un entraîneur.

7. Procédé selon l'une des revendications précédentes, caractérisé en ce qu'on utilise un ou plusieurs supports choisis dans le groupe des polymères, lipides, lécithines et cires.

8. Procédé selon l'une des revendications précédentes, caractérisé en ce que, en plus de la ou des substances actives et du ou des supports, on utilise des adjuvants, par exemple des agents tensio-actifs, et en ce que l'on récupère une préparation substance active/support/adjuvant.

9. Procédé selon l'une des revendications précédentes, caractérisé en ce qu'on utilise comme substance active un médicament, une toxine ou un virus, ou des fragments de virus.

10. Procédé selon l'une des revendications précédentes, caractérisé en ce que les mélanges substance active/support sont exempts de germes capables de se multiplier, après le traitement par le fluide gazeux.

11. Dispositif pour la mise en oeuvre du procédé selon l'une des revendications précédentes, caractérisé par
- une pompe à gaz liquéfié (P1),
- à chaque fois un évaporateur/échangeur de chaleur (W1) et (W2),
- une tour de pulvérisation (B3),
- un séparateur (A) pour la préparation substance active/support,
- un séparateur (B2) pour le milieu liquide facultatif,
- un réservoir de stockage (B1) pour le milieu liquide facultatif,
- un condenseur (K1) pour le fluide gazeux.
